# EUROPEAN PATENT APPLICATION

(11) **EP 4 825 368 A1**
(43) Date of publication of application: **21.10.2026**
(21) Application number: 25170257.7
(22) Date of filing: 14.04.2025
(51) Int. Cl.: A61K 8/44, A61K 8/46, A61K 8/60, A61Q 5/02, A61Q 19/10

(54) **PRESERVATIVE-FREE AND SULFATE FREE AQUEOUS SURFACTANT COMPOSITION**

(71) Applicant: BASF SE, 67056 Ludwigshafen am Rhein (DE)
(72) Inventor: BRUNN, Claudia, 40589 Düsseldorf-Holthausen (DE)
(74) Representative: BASF IP Association

(57) **Abstract**

A preservative-free and sulfate-free aqueous surfactant comprising
• one or more **alpha-sulfo fatty acid salts (A)** of the general formula (I),

R¹CH(SO₃M¹)COOM² (I)

in which the radical R¹ is a linear or branched alkyl or alkenyl radical with 6 to 16 carbon atoms and the radicals M¹ and M² - independently of one another - are selected from the group H, Li, Na, K, Ca, Mg, ammonium and alkanolamine, and encompass a disalt and/or a monosalt and/or a protonated acid, and
• one or more **alkyl glycosides (B)** of the general formula (II),

R²O-[G]ₚ (I)

in which R² is an alkyl and/or alkenyl radical with 8 to 18 carbon atoms, G is a sugar radical with 5 or 6 carbon atoms and p is numbers between 1 and 10,
• one or more **amidoalkylbetaines (C)** of the general formula (III),

R³-CO-NH-(CH₂)_{y}-N⁺(CH₃)₂-CH₂-COO⁻ (III)

in which the radical R³ is a linear or branched alkyl or alkenyl radical with 7 to 19 carbon atoms and the index y is an integer in the range 2 to 4 and the use of compound (A) as bacteriostatic surfactant for manufacturing preservative-free cosmetic products.

## Description

Personal care and cosmetic products particularly focusing on their role in cleaning such as body washes, shampoos, facial cleansers or liquid soaps are often based on aqueous compositions and have to contain preservatives. Preservatives are essential in aqueous cosmetic and personal care products to prolong their shelf life and prevent microbial growth. However, it is known that they can cause skin irritation and allergies, often in individuals with sensitive skin.

Especially traditional chemical preservatives have drawbacks in respect of their potential to cause skin irritation and allergies, and in addition for their environmental impact. Using natural preservatives like plant bactericides, results in limitations in view of their effectiveness and may complicate formulations due to incompatibilities.

Several alternative formulations that aim to reduce or eliminate the use of preservatives have therefore been proposed. Either they are using specific surfactant systems that provide both cleaning efficacy and mildness and still offer good stability and performance or they comprise specific additives such as a large amount of specific salts as described in EP 0 442 519 B2.

Liquid cleaning products often use sulfate-based surfactants because they are effective at creating a lot of foam, which is very stable and has improved cleaning capacity. However, sulfate-based surfactants, like sodium lauryl sulfate and sodium laureth sulfate, can as well be an irritant to mucosal membranes and are known to be harsher on hair and skin, especially colored hair, compared to formulations that are mostly free of sulfate-based surfactants.

Individuals recognize the broader implications of their choices, prompting them to pursue lifestyles, products and practices that benefit not only themselves but also the environment and society at large.

Therefore, there is a rapidly growing consumer demand for shampoo that is mostly free of sulfate-based surfactants. However, it can be challenging to use these surfactants in traditional liquid shampoos because it is difficult to create a formula that has acceptable lather volume, cleansing ability, stability, and clarity. To improve lather volume and cleansing, shampoos with sulfate-free surfactants usually have more surfactant than those with sulfate-based surfactants to achieve similar effectiveness. WO 2010/069500 describes hair cleansing compositions that are sulfate-free and produce voluminous and creamy foam. These compositions include at least two anionic surfactants with amid group.

In order to achieve an optimal balance between cleansing efficacy and mildness, allowing for regular use without causing skin or hair damage WO 2018/157974 discloses cosmetic cleansing compositions that effectively cleanse the skin and hair while maintaining mildness. The compositions are based on a specific surfactant system comprising anionic, amphoteric, and non-ionic surfactants, especially anionic surfactants such as disodium laureth sulfosuccinate and sodium cocoyl glutamate, amphoteric surfactants like cocamidopropyl betaine and sodium cocoamphoacetate and non-ionic surfactants including coco glucoside and lauryl glucoside. Nevertheless, the surfactant system requires the presence of preservatives such as phenoxyethanol and sodium benzoate.

Therefore it is an object of the current invention to provide aqueous surfactant compositions with sufficient cleaning capacity which are preservative- and sulfate-free in order to have an improved skin compatibility and ecotoxicology.

### Description of the Invention

The object of the invention was solved by a specific combination of surfactants comprising alpha-sulfo fatty acid salts as anionic surfactant which is a preservative-free and sulfate free aqueous surfactant composition comprising
- one or more **alpha-sulfo fatty acid salts (A)** of the general formula (I),

   R¹CH(SO₃M¹)COOM² (I)

   in which the radical R¹ is a linear or branched alkyl or alkenyl radical with 6 to 16 carbon atoms and the radicals M¹ and M² - independently of one another - are selected from the group H, Li, Na, K, Ca, Mg, ammonium and alkanolamine, and encompass a disalt and/or a monosalt and/or a protonated acid, and
- one or more **alkyl glycosides (B)** of the general formula (II),

   R²O-[G]ₚ (I)

   in which R² is an alkyl and/or alkenyl radical with 8 to 18 carbon atoms, G is a sugar radical with 5 or 6 carbon atoms and p is numbers between 1 and 10,
- one or more **amidoalkylbetaines (C)** of the general formula (III),

   R³-CO-NH-(CH₂)_{y}-N⁺(CH₃)₂-CH₂-COO⁻ (III)
in which the radical R³ is a linear or branched alkyl or alkenyl radical with 7 to 19 carbon atoms and the index y is an integer in the range 2 to 4.

Surprisingly this surfactant composition with a surfactant system comprising the components (A), (B) and (C) and alpha sulfo fatty acid salts as anionic surfactant fulfils the criteria of the preservation efficacy test for cosmetics according to DIN EN ISO 11930 although no preservative is added, the pH is slightly acid and even if the total amount of surfactants in the aqueous composition is low. Thus it provides an improved skin compatibility and improved ecotoxicology.

As used herein, "preservative-free" means the composition of the present invention is free of preservatives according to Annex V of the EU commission regulation for cosmetics No. 1223/2009.

Especially the aqueous composition is free of preservatives selected from the group consisting of ethanol, parabens, butylated hydroxytoluene (BHT), butylated hydroxyanisole (BHA), DMDM hydantoin, methylchloroisothiazolinone and methylisothiazolinone, chlorphenesin, phenoxyethanol, ethylhexylglycerin, benzyl alcohol, the following organic acids and alkali metal, alkalin earth metal, ammonium and monoethanolamine salts thereof as well as esters thereof: benzoic acid, sorbic acid, propionic acid, p-anisic acid, salicylic acid, dehydroacetic acid, formic acid

As used herein, "free of" in connection with preservative-free means less than 0.05 wt%, preferably less than 0.03 wt %, more preferably less than 0.01 wt % of all preservatives listed in Annex V of the EU commission regulation for cosmetics No. 1223/2009 based on the weight of the aqueous surfactant composition of the invention with the proviso that less than 5 % preferably less than 1 %, more preferred less than 0.001 % of the amount given in column g - maximum concentration in ready for use rinse-off preparation - of Annex V of the EU commission regulation for cosmetics No. 1223/2009 of each single listed preservative is added to the aqueous composition of the invention.

Most preferably no preservatives at all as listed in Annex V of the EU commission regulation for cosmetics No. 1223/2009 are added to the aqueous surfactant composition of the invention.

All percentages, parts and ratios are based upon the total weight of the compositions of the present invention, unless otherwise specified. All such weights as they pertain to listed ingredients are based on the active material and, therefore, do not include carriers or by-products that may be included in commercially available materials.

As used herein, "sulfate-free" means the composition of the present invention is free of sulfate-based surfactants, which are sulfate-group containing surfactants, characterized by the following chemical structure:
Rx-OSO₃⁻¹ , where Rx is a lipophilic group.

Preferably the inventive aqueous surfactant composition is free of alkyl sulfates and free of alkyl ether sulfates.

### The compounds (A)

The compounds (A), which are referred to within the context of the present invention as **alpha-sulfo fatty acid salts,** are obligatory for the aqueous surfactant compositions according to the invention. They have the aforementioned formula (I)

R¹CH(SO₃M¹)COOM² (I)

in which the radical R¹ is a linear or branched alkyl or alkenyl radical with 6 to 16 carbon atoms and the radicals M¹ and M² - independently of one another - are selected from the group H, Li, Na, K, Ca, Mg, ammonium and alkanolamines. Particularly preferred alkanolamines of the invention are Monoethanolamine, Diethanolamine, Triethanolamine and Mono-Isopropanolamine.

In the context of the present invention the compounds (A) named as "alpha-sulfo fatty acid salts" are defined by formula (I) in which the radicals M1 and M2 - independently of one another - are selected from the group H, Li, Na, K, Ca, Mg, ammonium and alkanolamines and thus encompass a disalt and/or a monosalt and/or a protonated acid.

With regard to the compounds (A), the proviso also applies that the fraction of the compounds (A) in which the radical R¹ is an alkyl or alkenyl radical with 14 or more carbon atoms - based on the total amount of the compounds (A) in the aqueous surfactant compositions - is 20% by weight or less.

In one embodiment, the proviso applies that the fraction of the compounds (A) in the aqueous surfactant compositions in which the radical R¹ is an alkenyl radical - based on the total amount of the compounds (A) - is 3% by weight or less.

In a preferred embodiment, the radical R1 in the formula (I) means a saturated, linear radical with 10 to 16 carbon atoms, where, with regard to the compounds (A), it is the case that the fraction of the compounds (A) in which the radical R1 is a decyl and/or a dodecyl radical - based on the total amount of the compounds (A) - is more than 70% by weight, preferably more than 80 % by weight, more preferably more than 90 % by weight and particular more than 95 % by weight.

Preferably, the radicals M¹ and M² in the formula (I) are Na.

The amount of compound (A) in the preservative- and sulfate-free aqueous composition is 0.1 to 10 wt %, preferably 0.5 to 5 wt %, mostly preferred 0.9 to 1.5 wt % active matter based on the weight of the preservative-free and sulfate-free aqueous surfactant composition. The presence of compound (A) is crucial for the microbiological stability of the inventive composition. Surprisingly surfactants systems of (A), (B) and (C) comprising only 1 wt % active matter of compound (A), which is 8 wt % based on the sum of surfactants (A), (B) and (C), is sufficiently stable to fulfil the acceptance criteria (log reduction) of the preservation efficacy test for cosmetics according to DIN EN ISO 11930.

A further subject matter of the invention is therefore the use of the aforementioned compound (A) as a bacteriostatic surfactant for manufacturing preservative-free cosmetic products. It was surprising that even at a very low concentration of 1 wt % active matter of (A) based on the aqueous surfactant composition of the invention the resulting composition could fulfil criteria A of the preservation efficacy test for cosmetics according to DIN EN ISO 11930 although no preservative is added. This was not given when adding sulfate-based surfactant as an anionic surfactant instead of (A) (Table 3c - comparison g, h).

The compounds (A) can be prepared by all methods known appropriately to the person skilled in the art. A particularly preferred method of preparation here is the sulfation of the corresponding carboxylic acids. Here, the corresponding carboxylic acid and in particular the corresponding fatty acids are reacted with gaseous sulfur trioxide, the sulfur trioxide being used preferably in an amount such that the molar ratio of SO₃ to fatty acid is in the range from 1.0: 1 to 1.1 : 1. The crude products obtained in this way, which are acidic sulfation products, are then partially or completely neutralized, preference being given to complete neutralization with aqueous NaOH. If desired, it is also possible to undertake purification steps and/or a bleaching (for adjusting the desired pale color of the products).

In a preferred embodiment it is the case that if the preservative- and sulfate-free aqueous surfactant composition comprises one or more ester sulfonates (E) of the general formula (V),

R⁵CH(SO₃M⁵)COOR⁶ (V)

in which the radical R⁵ is a linear or branched alkyl or alkenyl radical with 6 to 18 carbon atoms and the radical R⁶ is a linear or branched alkyl or alkenyl radical with 1 to 20 carbon atoms, where the radical R⁶ is an alkenyl radical or be branched only above 3 carbon atoms, and the radical M⁵ is selected from the group Li, Na, K, Ca, Mg, ammonium and alkanolamines, the weight of compounds (A) - based on the total weight of the compounds (A) and (E) - must be present to 50% by weight or more; preferably 90 % by weight and more.

### The compounds (B)

The compounds (B), which are referred to in the context of the present invention as **alkyl glycosides,** are obligatory for the aqueous surfactant compositions according to the invention. They have the aforementioned formula (II),

R²O-[G]ₚ (II)

in which R² is an alkyl and/or alkenyl radical with 8 to 22 carbon atoms, G is a sugar radical with 5 or 6 carbon atoms and p is numbers between 1 and 10.

Preferably the fraction of the compounds (B) in which the radical R² is an alkyl or alkenyl radical with 15 or more carbon atoms - based on the total amount of the compounds (B) in the aqueous surfactant compositions - is 5% by weight or less.

It may expressly be stated that the naming of the compounds (B) as alkyl glycosides - henceforth also referred to as APGs (singular: APG) - serves merely for a linguistically simple naming of the compounds (B) and should not be understood as being structurally limiting; hence in the definition according to the formula of the compounds (B) it is clarified that the radical R² can mean either an alkyl or an alkenyl radical and also - as the index p shows - that they can be alkyl or alkenyl oligoglycosides.

APGs of the form claimed here can be obtained by the relevant methods of preparative organic chemistry. The APGs can be derived from aldoses or ketoses with 5 or 6 carbon atoms. Preferably, the APGs are derived from glucose.

The index number p in the general formula (II) indicates the degree of oligomerization (DP degree = degree of polymerization). The degree of oligomerization of the APGs is between 1 and 10 and preferably between 1 and 6. Whereas p in an individual APG molecule must always be an integer and here in particular assumes the values in the range from 1 to 6, the value p for an APG which is a mixture of different APG molecules, which differ in their individual p values, is an analytically determined calculated parameter which in most cases is a fraction. Preferably, APGs are used with an average degree of oligomerization p in the range from 1.1 to 3.0. In this connection, preference is given in particular to those APGs whose average degree of oligomerization is less than 2 and is preferably in the range from 1.1 to 1.8 and in particular in the range from 1.2 to 1.7.

The average degree of oligomerization here is to be understood in the sense of how it is defined in the monograph K. Hill, W. von Rybinski, G. Stoll "Alkyl Polyglycosides. Technology, Properties and Applications" (VCH-Verlagsgesellschft, 1996) in the section "Degree of polymerization" (compare pages 11-12 of the book): there it reads "The average number of glycose units linked to an alcohol group is described as the (average) degree of polymerization (DP)." In explanatory figure 2, which describes a typical distribution of dodecyl glycoside oligomers of an AOPG with a degree of DP of 1.3, the average degree of DP is also described by a corresponding mathematical formula.

The alkyl radical R² can be derived from primary alcohols having 6 to 22, preferably 6 to 18 carbon atoms. The alkyl radical R² can also be derived from hexyl-, octyl-, decyl-alcohol or preferably lauryl alcohol, myristyl alcohol, cetyl alcohol, palmoleyl alcohol, stearyl alcohol, isostearyl alcohol, oleyl alcohol, elaidyl alcohol, petroselinyl alcohol, arachyl alcohol, gadoleyl alcohol, behenyl alcohol, erucyl alcohol, brassidyl alcohol and also technical grade mixtures thereof. The radical R² is more preferably derived from primary alcohols with 8 to 12 carbon atoms, as are obtained for example in the hydrogenation of technical-grade fatty acid methyl esters or in the course of the hydrogenation of aldehydes during Roelen oxo synthesis.

Preference is given to APGs which are derived from glucose and in which the radical R² is a saturated alkyl radical with 8 to 12 carbon atoms and which have an average degree of oligomerization in the range from 1.1 to 3.0 and in particular in the range from 1.2 to 1.8 and particularly preferably in the range from 1.2 to 1.7. These APGs can for example be prepared by reacting a sugar, in particular glucose, under acid catalysis with a fatty alcohol mixture, the fatty acid mixture used preferably being a forerunning produced during the distillative separation of technical-grade C₈₋₁₈-coconut fatty alcohol, which comprises predominantly octanol-1 and decanol-1 and also small amounts of dodecanol-1.

The amount of compound (B) in the preservative- and sulfate-free aqueous composition is 0.1 to 20 wt %, preferably 3 to 12 wt %, mostly preferred 5 to 9 wt % active matter based on the weight of the preservative-free and sulfate-free aqueous surfactant composition.

### The compounds (C)

The compounds (C), which are referred to in the context of the present invention as **amidoalkylbetaines,** are obligatory for the aqueous surfactant compositions according to the invention. They have the aforementioned formula (III)

R³-CO-NH-(CH₂)_{y}-N⁺(CH₃)₂-CH₂-COO⁻ (III)

in which the radical R³ is a linear or branched alkyl or alkenyl radical with 7 to 19 carbon atoms and the index y is an integer in the range 2 to 4. Also applicable - as likewise stated above - is the proviso that the fraction of the compounds (C) in which the radical R² is an alkenyl radical - based on the total amount of the compounds (C) in the aqueous surfactant compositions - is 3% by weight or less.

The compounds (C) can be prepared by all of the methods known appropriately to the person skilled in the art.

In one embodiment, the index y in the formula (III) is the number 3.

In one embodiment, radical R² in the formula (III) is a saturated, linear radical with 11 to 17 carbon atoms where, with regard to the compounds (C), it is the case that the fraction of the compounds (C) in which the radical R² is an undecyl or a tridecyl radical - based on the total amount of the compounds (C) - is 60% by weight or more.

In a preferred composition, the compounds (C) are cocamidopropylbetaine. It is an industrially available product which is typically produced in two steps:
Firstly, coconut fatty acid is reacted with dimethylaminopropylamine (DMAPA, chemical formula NH₂-(CH₂)₃-N(CH₃)₂). The resulting amide here is then reacted in a second step with sodium chloroacetate (chemical formula Cl-CH₂-COONa) in the presence of NaOH, a quaternization taking place with the elimination of NaCl. The product of technical grade thus obtainable can comprise, besides cocamidopropylbetaine and NaCl, as a consequence of production, as byproducts, glycerol, partial glycerides, glycolic acid, diglycolic acid and free fatty acid, it being possible to reduce the content of these byproducts through the choice of suitable production conditions. If desired, these byproducts can also be further reduced in their content or be eliminated entirely by means of additional purification steps purification.

The amount of compound (C) in the preservative- and sulfate-free aqueous composition is 0.5 to 15 wt %, preferably 2 to 10 wt %, mostly preferred 3.5 to 5 wt % active matter based on the weight of the preservative-free and sulfate-free aqueous surfactant composition.

The preservative-free and sulfate free aqueous surfactant according to the invention contains compound (A) at an amount of 0.1 to 10 wt%, compound (B) at an amount of 0.1 to a 20 wt% and compound (C) at an amount of 0.5 to 15 wt% based on the total weight of the preservative-free and sulfate-free aqueous surfactant composition.

Preferably the preservative-free and sulfate free aqueous surfactant according to the invention contains compound (A) at an amount of 0.5 to 5 wt%, compound (B) at an amount of 3 to a 12 wt% and compound (C) at an amount of 2 to 10 wt% based on the total weight of the preservative-free and sulfate-free aqueous surfactant composition.

Mostly preferred the preservative-free and sulfate free aqueous surfactant contains compound (A) at an amount of 0.9 to 1.5 wt%, compound (B) at an amount of 5.0 to 9.0 wt% and compound (C) at an amount of 3.5 to 5.0 wt% based on the total weight of the preservative-free and sulfate-free aqueous surfactant composition.

The preservative-free and sulfate-free aqueous surfactant composition may contain further surfactants provided that anionic sulfate-based surfactants are excluded, specifically alkyl sulfates and alkylether sulfates are excluded.

Preferably the total amount of surfactants of compounds (A) and (B) and (C) in the preservative-free and sulfate-free aqueous surfactant composition is 3 to 30 wt %, more preferred 8 to 20 wt %, mostly preferred 10 to 15 wt % active matter based on the weight of the preservative-free and sulfate- free aqueous surfactant composition.

More preferable the total amount of surfactants in general, of compounds (A) and (B) and (C) and further surfactants, in the preservative-free and sulfate-free aqueous surfactant composition is 3 to 30 wt %, more preferred 8 to 20 wt %, mostly preferred 10 to 15 wt % active matter based on the weight of the preservative-free and sulfate- free aqueous surfactant composition.

It is surprising that also aqueous surfactant compositions with a low active matter content of 10 to 15 wt % of the surfactants (A) and (B) and (C) - without other surfactants - fulfil the criteria A of the preservation efficacy test for cosmetics according to DIN EN ISO 11930 although no preservative is added.

In one embodiment of the invention, the inventive preservative-free compositions could comprise further surfactants.

Surfactants are amphiphilic substances which can dissolve organic, nonpolar substances in water. They cause, as a result of their specific molecular structure with at least one hydrophilic and a hydrophobic molecular moiety, a lowering of the surface tension of the water, the wetting of the skin, the facilitation of soil removal and dissolution, easy rinse-off and - if desired - foam regulation.

Further surface-active substances present may be anionic, nonionic, cationic and/or amphoteric or zwitterionic surfactants.

Typical examples of these groups are :
Anionic surfactants: Sodium methyl cocoyl taurate, sodium lauryl sarcosinate, alkyl benzene sulfonates, alkanesulfonates, α-olefin sulfonates, alkyl ether sulfonates, glycerol ether sulfonates, α-methyl ester sulfonates, sulfo fatty acids, mono- and dialkyl sulfosuccinates, mono- and dialkyl sulfosuccinamates, sulfotriglycerides, amide soaps, ether carboxylic acids and salts thereof, fatty acid isethionates, fatty acid sarcosinates, fatty acid taurides, N-acylamino acids (e.g., acyl lactylates, acyl tartrates, acyl glutamates, acyl aspartates), protein fatty acid condensates (particularly wheat-based vegetable products), alkyl (ether) phosphates.

Non-ionic surfactants: Fatty acid alkanolamides, ethylene glycol stearate, ethylene glycol distearate, sucroglycerides, ethoxylated fatty alcohols (derived from lauryl, cetylstearyl, stearyl, cetyl, and oleocetyl alcohols), fatty alcohol polyglycol ethers, alkylphenol polyglycol ethers, fatty acid polyglycol esters, fatty acid amide polyglycol ethers, fatty amine polyglycol ethers, alkoxylated triglycerides, mixed ethers and mixed formals, polyglycerol fatty acid esters, fatty acid-N-alkyl glucamides, protein hydrolyzates (particularly wheat-based vegetable products), polyol fatty acid esters, sugar esters, sorbitan esters, polysorbates, amine oxides.

Cationic surfactants: Tertiary amine salts, mono alkyl trimethyl ammonium chloride, mono alkyl trimethyl ammonium methyl sulphate, dialkyl dimethyl ammonium chloride, dialkyl dimethyl ammonium methyl sulphate, trialkyl methyl ammonium chloride, quaternary ammonium compounds (e.g., dimethyl distearyl ammonium chloride), esterquats (quaternized fatty acid trialkanolamine ester salts), ammonium halides (e.g., chlorides and bromides), cetyltrimethylammonium chloride, stearyltrimethylammonium chloride, distearyldimethylammonium chloride, lauryldimethylammonium chloride, lauryldimethylbenzylammonium chloride, tricetylmethylammonium chloride, quaternized protein hydrolyzates.

Amphoteric or zwitterionic surfactants: Betaines such as component (C) (e.g., alkyl betaines, alkylamidopropyl betaines, alkylamidopropyl hydroxy sultaines), alkylampho acetates, alkylamphodiacetates, alkylamphopropionates, alkylamphodipropionates, alkyliminodipropionates, alkyliminodiacetate, aminopropionates, aminoglycinates, imidazolinium betaines, sulfobetaines, mono- and/or dialkyl sulfosuccinates, fatty acid isethionates, fatty acid sarcosinates, fatty acid taurides, fatty acid glutamates, α-olefin sulfonates, ether carboxylic acids, alkyl oligoglucosides, fatty acid glucamides, amphoacetals, protein fatty acid condensates (preferably based on wheat proteins), N-alkylglycines, N-alkylpropionic acids, N-alkylaminobutyric acids, N-alkylimino¬dipropionic acids, N-hydroxyethyl-N-alkylamidopropyl¬glycines, N-alkyltaurines, N-alkylsarcosines, 2-alkyl¬aminopropionic acids, alkylaminoacetic acids, N-cocoalkylaminopropionate, cocoacylaminoethyl-aminopropionate, C12-18-acylsarcosine.

The preservative-free aqueous surfactant composition of the invention may contain amounts of up to 10 wt %, preferably up to 5 wt%, more preferably up to 3 wt % based on the preservative-free aqueous surfactant composition of other surfactants than compounds (A), (B) or (C) provided they are sulfate-free. Mostly preferred the aqueous surfactant composition of the invention does not contain other surfactants than compounds (A), (B) and (C).

In one preferred embodiment, the **pH** of the aqueous surfactant composition according to the invention is in the range of 3.8 to 6.0, preferably 4.0 to 5.5, mostly preferred 4.5 to 5.2.

The **viscosity** of the aqueous surfactant compositions - measured using a Brookfield DV3+ laboratory rheometer at 23°C, 10 rpm, spindle no. 4- is preferably 1000 mPas or higher. The viscosity is achieved just by micellar thickening of the surfactant system without the addition of any thickener, like polymer or assoziative thickeners.

The preservative- and sulfate-free aqueous surfactant composition of the invention has a good cleaning capacity which is due to the good foaming ability and good foam stability. A large foam volume has been measured for the combination of components (A), (B) and (C) although no sulfate-based surfactants have been added

### Use of the compositions

A further subject matter of the invention is the use of the aforementioned composition for preservative- and sulfate-free surfactant based liquid compositions especially cosmetic products. With regard to cosmetic products, particular preference is given here especially to those which are present in the form of hair shampoos, conditioners, hair colorants, shower gels, soaps, syndets, liquid hand cleansers, facial cleansers, washing pastes, washing lotions, scrub preparations, foam baths, oil baths, shower baths, shaving foams, shaving lotions and shaving creams, for home care compositions laundry detergents and dish detergent are preferred.

Besides the high dermatological compatibility (e.g. with skin, oral and ocular mucuous membrane) due to the absence of preservatives and sulfate-based surfactants and the improved (eco-)toxicological profile despite the good foaming ability and good foam stability there are are many additional objects to provide an improved surfactant composition for personal care and cosmetics solved. The aqueous surfactant compositions of the current invention has several additional advantages such as:
- a good sustainability profile for the components (A), (B) and (C), which can be sourced from natural, renewable raw materials and being biodegradable, having a certain content of renewable carbon
- a good compatibility with other ingredients and auxiliaries in a formulation due to the absence of preservatives and sulfate-based surfactants
- easy and energy-saving manufacturing process as the composition can be prepared by hot or cold process
- good stability against decomposition over a wide temperature and pH range below 6,
- good thickenability, e.g. being capable of micellar thickening to create viscosity
   as known from WO2019/169100 sulfate-free cleaning compositions are difficult to thicken sufficiently to afford the good sensory properties and two approaches such as high levels of non-sulfate surfactants or high levels of rheology modifiers are employed to thicken sulfate-free cleanser formulas. Surprisingly the composition of the current invention has sufficient viscosity without high amounts of surfactants or rheology modifiers.

### Examples

### Aqueous surfactant compositions

Aqueous surfactant compositions according to Tables 3a-c are prepared by adding the respective components (Texapon SFA UP Powder, Dehyton PK 45, Plantacare 1200 UP, Lamesoft PO 65, Texapon NSO UP, Texapon K30 UP, source: BASF SE, Ludwigshafen) to demineralized water and mixing until completely dissolved and clear. To speed up the process and to dissolve solid components the aqueous solution can be heated to approx. 50°C. After cooling down the pH value is adjusted to 5,0 +/- 0,1 with citric acid (50% sol.).

### Preservative efficacy test

The preservative efficacy test to evaluate the antimicrobial efficacy of the compositions according to the invention was conducted according to ISO EN 11930, which is an international standard method used to investigate the general antimicrobial protection of cosmetic products. It ensures that cosmetic products are free from microbial contamination throughout their shelf-life by assessing the effectiveness of their preservative systems.

The primary organisms included in the testing process are:
Staphylococcus aureus ATCC 6538: A gram-positive bacterium known for its potential to cause skin infections.
Pseudomonas paraeruginosa (formerly aeruginosa) ATCC 9027: A gram-negative bacterium that can lead to serious infections, particularly in immunocompromised individuals.
Escherichia coli ATCC 8739: Another gram-negative bacterium often associated with contamination and foodborne illnesses.
Candida albicans ATCC 10231: A yeast that can cause infections, especially in individuals with weakened immune systems.
Aspergillus brasiliensis ATCC 16404: A fungus commonly found in soil and is a frequent food contaminant which can cause lung infections in people with weakened immune system..
The key procedure involves the following:
The product is inoculated with known concentration of each of the test microorganisms. The initial microbial concentration ranges from 10^5 to 10^6 CFU/mL or g (colony-forming units per milliliter or gram). Following inoculation, the product is incubated at controlled environmental conditions usually at 20-25°C. This allows microorganisms which are in contact with the preservative system of the product to grow. At defined time points, the microbial counts are obtained to assess the decrease in microbial load. Typically, the following time points are employed.

T0: immediately after inoculation, T7: on day 7, T14: on day 14, T28: on day 28

The product is evaluated based on the reduction of microbial load over time while being subjected to the following criteria:
For Bacteria: A ≥ 3-log reduction (99.9%) of CFU at day 7, with no subsequent increase at day 28.
For Yeast and Mold: A ≥ 1-log reduction (90%) in CFU at day 7, with no subsequent increase at day 28.

The result of the challenge test is verified to ensure continued adequate preservation of the product over time. If microbial criteria are not achieved, reformulation or improvement of the preservative system may be required.

**Table 1: Evaluation and acceptance criteria (log reduction) according to DIN EN ISO 11930 (a difference of 0.5 log units is allowed)**

| | **0 h** | **7 d** | **14 d** | **28 d** |
|---|---|---|---|---|
| Bacteria (Criteria A) | --- | ≥ 3,0 | ≥ 3,0 * | ≥ 3,0 * |
| Bacteria (Criteria B) | --- | --- | ≥ 3,0 | ≥ 3,0 * |
| Candida albicans (Criteria A) | --- | ≥ 1,0 | ≥ 1,0 * | ≥ 1,0 * |
| Candida albicans (Criteria B) | --- | --- | ≥ 1,0 | ≥ 1,0 * |
| Aspergillus brasiliensis (Criteria A) | --- | --- | ≥ 0,0 | ≥ 1,0 * |
| Aspergillus brasiliensis (Criteria B) | --- | --- | ≥ 0,0 | ≥ 0,0* |

| | | | | |
|---|---|---|---|---|
| * no increase since last count | | | | |

**Foam test:** To test the initial foaming behavior (rotor foam method), a standard commercial measuring instrument was used (Sita Foam Expert System). For this, an aqueous surfactant solution was first prepared as follows: 10 g/L of each aqueous surfactant composition to be tested were dissolved completely in hard water, which was adjusted to 15°dH before (A degree of 'German hardness (°dH, deutsche Härte))' is defined as 10 mg/L CaO or 17.848 ppm.). The solution prepared in this way was heated to 30°C.

250 ml of the heated solution were transferred into the measuring vessel and foamed at a speed of 1300 rotations per minute for 10 seconds, the foam volume then present was measured (in ml), then foamed by stirring for a further 10 seconds, the foam volume then present was measured (in ml), etc., i.e. the foam volume is determined every 10 seconds during foaming. After a foaming time of 180 seconds, the measurement ended. The measurement was repeated for each sample 3 times, in each case using a fresh solution from the same batch, and the result of the measurements after 40, 60 and 80 seconds was given as an average from these three measurements.

It could be detected that the foaming properties of the surfactant composition according to the invention are comparable or improved in view of usual surfactant compositions comprising sulfate containing surfactants.

**Viscosity testing:** Viscosity was measured using a Brookfield DV3+ laboratory rheometer at 23°C, 10 rpm, spindle no. 4.

### EPT Epicutaneous Patch Test:

The EPT is a method to ascertain the dermal irritation potential of a substance following a single application of the test substance onto the back of a volunteer. It allows skin compatibility to be assessed under the exaggerated conditions of occlusivity. The parameters erythema, edema, squamation and fissure are assessed 6 h, 24 h, 48 h and 72 h after patch removal (Walker et al., 1996). The scores are then used to calculate the total irritation score. Prior to testing the products were adjusted to 2% AS. A clear-cut grading scheme for the irritation potential in human tests has not yet been established as the protocols have not been standardized, assessment of the irritation effects and controls differ depending on the laboratory conducting the test and the reactions of volunteers being tested. Therefore, classification of the irritation potential was defined following a non-validated grading based on a relative irritation scores (relative to SDS, Sodium Dodecyl Sulfate, tested at 0,5%): >75%: severely irritating; >50%: irritating; >25%: moderately irritating; >10%: slightly, <=10%: very slightly irritating.

The irritation potential of the surfactant composition according to the invention (inv a of table 3a) was very low as the EPT result relative score to SDS tested at 0,5% gave a value of 5,4%, which is considered as "very slightly irritating".

### Results

### Preservation efficacy test (ISO 11930:2022-08)-

Table 2: Development of the number of microorganisms at 20-25°C after inoculation of the sample of the inventive composition a of Table 3a with the described microorganisms.

| **Analysis** | **0 h** | **7 d** | **14 d** | **28 d** |
|---|---|---|---|---|
| Pseudomonas aeruginosa/ Pseudomonas paraeruginosa ATCC 9027 | 4,6 x 10^5 | < 1,0 x 10^1 | < 1,0 x 10^1 | < 1,0 x 10^1 |
| Log reduction | --- | > 4,7 | > 4,7 | > 4,7 |
| Staphylococcus aureus ATCC 6538 | 2,4 x 10^5 | < 1,0 x 10^1 | < 1,0 x 10^1 | < 1,0 x 10^1 |
| Log reduction | --- | > 4,4 | > 4,4 | > 4,4 |
| Escherichia coli ATCC 8739 | 3,2 x 10^5 | < 1,0 x 10^1 | < 1,0 x 10^1 | < 1,0 x 10^1 |
| Log reduction | --- | > 4,5 | > 4,5 | > 4,5 |
| Candida albicans ATCC 10231 | 3,9 x 10^4 | 1,0 x 10^3 | 2,2 x 10^2 | < 1,0 x 10^1 |
| Log reduction | --- | 1,6 | 2,2 | > 3,6 |
| Aspergillus brasiliensis ATCC 16404 | 4,5 x 10^4 | --- | 1,5 x 10^5 | 5,0 x 10^3 |
| Log reduction | --- | --- | -0,5 increase | 1,0 |

| | | | | |
|---|---|---|---|---|
| All results of microbial counts are reported in cfu/g or ml (cfu = colony forming units) | | | | |

**Table 3 a - Aqueous surfactant compositions according to the invention**

| | Inv a | Inv b | Inv c |
|---|---|---|---|
| **INCI Component** | **wt% active matter** | **wt% active matter** | **wt% active matter** |
| Disodium 2-Sulfolaurate | 1.0 | 1.0 | 1.0 |
| Lauryl Glucoside | 6.9 | 6.9 | 6.9 |
| Cocamidopropyl Betaine | 4.1 | 4.1 | 4.1 |
| Glyceryl Oleate | | 0.5 | 0.5 |
| Coco Glucoside | | 0.5 | 0.5 |
| Perfume Green Vanilla (Düllberg) | | | 0.3 |
| Total amount of surfactants | 12.0 | 12.5 | .12.5 |
| | | | |

| **Challenge Test** | **A criteria** | **A criteria** | **A criteria** |
|---|---|---|---|
| viscosity [mPas] | 7400 | 1020 | 1080 |
| Appearance | clear | clear | clear |

**Table 3b: Single Surfactant Compositions (non-inventive)**

| | Non Inv d | Non Inv e | Non Inv f |
|---|---|---|---|
| **Surfactant Component** | **wt% active matter** | **wt% active matter** | **wt% active matter** |
| Disodium 2-Sulfolaurate | | 12.0 | |
| Lauryl Glucoside | 12.0 | | |
| Cocamidopropyl Betaine | | | 12.0 |
| Total amount of surfactants | 12.0 | 12.0 | 12.0 |
| | | | |

| **Challenge Test** | **doesn't comply** | **A criteria** | **doesn't comply** |
|---|---|---|---|
| viscosity [mPas] | <600 | <200 | <200 |
| Appearance | clear | turbid | clear |

**Table 3 c - Aqueous surfactant compositions -comparison without (A) - non-inventive**

| **Surfactant Component** | **wt% active matter** | **wt% active matter** | **wt% active matter** |
|---|---|---|---|
| Disodium 2-Sulfolaurate | - | - | - |
| Lauryl Glucoside | 6.9 | 6.9 | 7.55 |
| Cocamidopropyl Betaine | 4.1 | 4.1 | 4.45 |
| Sodium Laureth Sulfate | 1.0 | - | - |
| Sodium Coco-Sulfate | - | 1.0 | - |
| Total amount of surfactants | 12.0 | 12.0 | 12.0 |
| | | | |

| **Challenge Test** | **doesn't comply** | **doesn't comply** | **doesn't comply** |
|---|---|---|---|
| Appearance | clear | clear | clear |

## Claims

1. A preservative-free and sulfate-free aqueous surfactant comprising
• one or more **alpha-sulfo fatty acid salts (A)** of the general formula (I),
R¹CH(SO₃M¹)COOM² (I)
in which the radical R¹ is a linear or branched alkyl or alkenyl radical with 6 to 16 carbon atoms and the radicals M¹ and M² - independently of one another - are selected from the group H, Li, Na, K, Ca, Mg, ammonium and alkanolamine, and encompass a disalt and/or a monosalt and/or a protonated acid, and
• one or more **alkyl glycosides (B)** of the general formula (II),
R²O-[G]ₚ (I)
in which R² is an alkyl and/or alkenyl radical with 8 to 18 carbon atoms, G is a sugar radical with 5 or 6 carbon atoms and p is numbers between 1 and 10,
• one or more **amidoalkylbetaines (C)** of the general formula (III),
R³-CO-NH-(CH₂)_{y}-N⁺(CH₃)₂-CH₂-COO⁻ (III)
in which the radical R³ is a linear or branched alkyl or alkenyl radical with 7 to 19 carbon atoms and the index y is an integer in the range 2 to 4.

2. The composition according to claim 1, where the radicals M¹ and M² are Na.

3. The composition according to any one of claims 1 or 4, wherein the pH of the composition is in the range from 3.8 to 6.

4. The preservative-free and sulfate-free aqueous surfactant according to any of claims 1 to 6, **characterized in that** it contains compound (A) at an amount of 0.1 to 10 wt%, compound (B) in an amount of 0.1 to a 20 wt% and compound (C) in an amount of 0.5 to 15 wt% with the weight percentages based on the total weight of the preservative-free and sulfate-free aqueous surfactant composition.

5. The preservative-free and sulfate free aqueous surfactant composition according to any of claims 1 to 4, **characterized in that** it contains compound (A) at an amount of 0.9 to 1.5 wt%, compound (B) in an amount of 5.0 to 9.0 wt% and compound (C) in an amount of 3.5 to 5.0 wt% with the weight percentages based on the total weight of the preservative-free and sulfate-free aqueous surfactant composition.

6. The preservative-free and sulfate-free aqueous surfactant composition according to any of claims 1 to 4, **characterized in that** the total amount of surfactants, the sum of compounds (A) and (B) and (C) and optionally further surfactants, in the preservative-free and sulfate-free aqueous surfactant composition is 3 to 30 wt active matter based on the weight of the preservative-free and sulfate- free aqueous surfactant composition.

7. The use of compound (A) as bacteriostatic surfactant for manufacturing preservative-free cosmetic products.

8. The use of the composition according to any of claims 1 to 6 for preservative- and sulfate-free cosmetic products in the form of hair shampoos, conditioners, hair colorants, shower gels, soaps, syndets, liquid hand cleansers, facial cleansers, washing pastes, washing lotions, scrub preparations, foam baths, oil baths, shower baths, shaving foams, shaving lotions and shaving creams.
